# EUROPEAN PATENT SPECIFICATION

(11) **EP 3 389 795 B1**
(45) Date of publication and mention of the grant of the patent: **01.12.2021**
(21) Application number: 15910913.1
(22) Date of filing: 16.12.2015
(51) Int. Cl.: A61K 8/24, A61K 8/81, A61Q 15/00, C08K 5/19, C01B 25/40

(54) **OCCLUSIVE PERSONAL CARE COMPOSITION**
VERSCHLIESSENDE KÖRPERPFLEGEZUSAMMENSETZUNG
COMPOSITION D'HYGIÈNE PERSONNELLE OCCLUSIVE

(43) Date of publication of application: 24.10.2018
(73) Proprietor: Colgate-Palmolive Company, New York, NY 10022 (US)
(72) Inventor: DUBOVOY, Viktor, Cresskill New Jersey 07626 (US); STRANICK, Michael, Bridgewater New Jersey 08807 (US); HILLIARD, Peter, Far Hills New Jersey 07931 (US); DU-THUMM, Laurence, Princeton New Jersey 08540 (US); PAN, Long, Somerset New Jersey 08873 (US)
(74) Representative: Wichmann, Hendrik
(86) International application number: PCT/US2015/066100
(87) International publication number: WO 2017/105438

(56) References cited:
- EP-A2- 0 353 648
- WO-A1-02/074271
- WO-A1-2010/070140
- WO-A1-2013/013903
- FR-A1- 2 871 376
- US-A1- 2012 021 025
- US-A1- 2015 306 008
- CINI ET AL.: 'Polyphosphates as inorganic polyelectrolytes interactingwith oppositely charged ions, polymers and deposited on surfaces: fundamentals and applications'.' ADVANCES IN COLLOID AND INTERFACE SCIENCE . vol. 209, 2014, pages 89 - 97, XP055391244
- None

## Description

### BACKGROUND

There have been many antiperspirants designed to help people reduce sweat. Examples of these can be found in U.S. Code of Regulation 21 C.F.R. § 350. The majority of the active agents used in antiperspirants are aluminum and zirconium halide compounds and complexes and their derivatives. While generally effective, some of these compounds are acidic and can stain clothes, among other things. Thus, there is a need for alternative antiperspirant actives. Embodiments of the present invention are designed to meet this need. EP 0 353 648 A2 discloses oral compositions and a method for inhibiting tartar on teeth, wherein said compositions comprise a fluoride source, a dental abrasive, and an antihydrolysis agent selected from homopolymers of substituted acrylamides, homopolymers of unsaturated sulfonic acids and salts thereof, and homopolymers and copolymers of cationic monomers. WO 2010/070140 A1 discloses an antiperspirant complex formed from a cosmetic composition A and a cosmetic composition B different from composition A, the said composition A comprising one or more compounds capable of reacting with one or more compounds of the cosmetic composition B by forming one or more ionic bonds to give an antiperspirant effect.

### SUMMARY

An embodiment of the present disclosure is directed to a personal care composition as defined in claim 1.

In another aspect, the present disclosure provides a method of occluding pores of a subject comprising applying a personal care composition to the skin of the subject.

Further areas of applicability of the present disclosure will become apparent from the detailed description provided hereinafter.

### DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

In one embodiment, the present disclosure provides a soluble quaternary ammonium polymer/polyphosphate complex. The complex may be made by combining in a composition at least one polyphosphate compound having 2 or more phosphate units and at least one quaternary ammonium polymer. These ingredients may be combined to form a pre-formed complex, which could be prepared in bulk, and then incorporated into personal care compositions. Such a combination may occurs in a suitable solvent, such as an aqueous solvent or non-aqueous solvent (e.g., hydrophilic or hydrophobic). Alternatively, these ingredients may be combined during the manufacture of a personal care composition, in order to form the complex in-situ. The combining of the materials can be in an aqueous solvent.

In one embodiment, the quaternary ammonium polymer is a poly(N-(trialkylaminoalkyl)acrylamide), for example, [CH₂CHCONHR]ₙ, wherein R is a quaternary ammonium trialkylaminoalkyl group. Such trialkyl aminoalkyl groups are of the general formula (alkylene)N⁺(alkyl)₃X⁻., wherein X is a counterion to the quaternary ammonium ion. "Alkylene" may be a C2-10 straight or branched chain alkyl moiety, while "alkyl" may be a C1-8 straight or branched chain alkyl moiety. "X" may be any suitable anion, such as fluoride, chloride, bromide, sulfate, phosphate, and the like. Such group include, for example, trimethylaminoethyl, trimethylaminopropyl, triethylaminoethyl, triethylaminopropyl, trimethylaminobutyl, triethylaminnobutyl, and the like. In one embodiment, the trialkyl aminoalkyl group is trimethylaminopropyl, e.g., trimethylaminopropyl chloride. Such polymer is commonly known as polyacrylamidopropyltrimonium chloride. The polyacrylamidopropyltrimonium chloride can be purchased as N-Durance A1000 from Ashland Chemical. In one embodiment, the number average molecular weight is 100,000 to 500,000 daltons, 200,000 to 400,000 daltons, or about 300,000 daltons.

Any polyphosphate having 2 or more phosphate units can be employed as the at least one polyphosphate, in any salt form. In one embodiment, the polyphosphate comprises a tripolyphosphate, such as sodium tripolyphosphate or potassium tripolyphosphate, or a diphosphate (pyrophosphate), such as tetrasodium pyrophosphate or tetrapotassium pyrophosphate. In another embodiment, the polyphosphate may comprises polyphosphates having 4 or more or 6 or more phosphate units. In a particular embodiment, the polyphosphate comprises a long chain polyphosphate having 6 to 50 phosphate units, e.g., 6 to 30 phosphate units, 10-30 phosphate units or 20-30 phosphate units. One such polyphosphate is sodium hexametaphosphate, which is a 6-phosphate cyclic molecule of the formula (NaPO₃)₆. Linear 6-phosphate polyphosphate may also be used. The polyphosphate used herein may be either linear, cyclic or mixtures of both forms. The counter ion for the polyphosphate can be any suitable cation. Examples include, but are not limited to, sodium, potassium, calcium, magnesium, ammonium, zinc and the like. Particular examples of long-chain polyphosphates include those having an average of 6, 9, 11, 12, 13, 15, 17, 18, 19, 21, 23, 24 or 25 phosphate units. A preferred polyphosphate is a polyphosphate having an average length of 20-25 phosphate units, e.g., 23 phosphate units. In one embodiment, the polyphosphate comprises a sodium polyphosphate having an average length of 20-25 phosphate units, e.g., 23 phosphate units. Such a polymer is often referred to as "sodium hexametaphosphate, long chain,", and has also been referred to as sodium polyphosphate, sodium polymetaphosphate, glassy sodium polyphosphate, glassy sodium phosphate, Glass H and Vitrafos. This polyphosphate typically has a P₂O₅ content of 68-71 wt%, e.g., about 69 wt%. To avoid confusion, as used herein below, the term "sodium polymetaphosphate" or "SPMP" refers to a sodium polyphosphate of average chain length 20-25 phosphate units, e.g., 23 phosphate units, either linear, cyclic or a mixture. Likewise, the term "polymetaphosphate" refers to a polyphosphate of average chain length of 20-25 phosphate units, e.g. 23 phosphate units, either linear, cyclic or a mixture.

The phosphorus to polymer ratio can be any ratio that results in a soluble complex at the desired pH in an aqueous solution. The ratio of polyphosphate to polymer is from 1:2 to 50:1, by weight, for example, 1:1 to 10:1, or 1:1 to 9:1, or 1:1 to 8:1, or 2:1 to 8:1, or 2:1 to 6:1, or 2:1 to 5:1, or 2:1 to 4:1, or 2:1 to 3:1, or 10:1 to 50:1, or 10:1 to 40:1 or 10:1 to 25:1 or 10:1 to 25:1, or 12:1 to 24:1, or 16:1 to 20:1, or about 2:1, or about 2.5:1 or about 3:1, or about 12:1 or about 16:1 or about 20:1, by weight.

The compositions of the present disclosure can be included in a personal care composition. Examples of such compositions include, but are not limited to, antiperspirants, deodorants, body washes, shower gels, bar soaps, shampoo, hair conditioners and cosmetics.

For antiperspirant and/or deodorant compositions, the carrier can be any carrier that is used for antiperspirants and/or deodorants. The carrier can be in the form of a stick, a gel, a roll-on, or an aerosol (e.g. spray). For stick formulations, the carrier may include oils and/or silicones and gelling agents.

In an embodiment, the personal care compositions, such as antiperspirants and/or deodorants, include the soluble quaternary ammonium polymer/polyphosphate complex made by combining in the composition at least one quaternary ammonium polymer and at least one polyphosphate having 2 or more phosphate units. These ingredients may be combined to form a pre-formed complex, which could be prepared in bulk, and then incorporated into personal care compositions. Such a combination may occurs in a suitable solvent, such as an aqueous solvent or non-aqueous solvent (e.g., hydrophilic or hydrophobic). Alternatively, these ingredients may be combined during the manufacture of a personal care composition, in order to form the complex in-situ. The combining of the materials can be in an aqueous solvent.

In an embodiment, the complex can also be used to enhance the efficacy of other antiperspirant salts comprising a polyvalent cation, for example antiperspirant complexes of (i) aluminum and optionally zirconium, (ii) chlorohydrate, and (iii) optionally an amino acid and/or ammonium acid, for example glycine and/or trimethylglycine, e.g., aluminum zirconium tetrachlorohydrex glycine. In an embodiment, these other antiperspirant salts can be added to the formulations of the present disclosure in addition to the quaternary ammonium polymer polyphosphate complex. In an alternative embodiment, the formulation includes only very small amounts or is entirely or substantially free of the above aluminum or zirconium antiperspirant active complexes. For example, the formulations can include less than 2 weight %, or less than 0.5 weight %, or less than 0.1 weight %, or less than 0.01 weight %, or less than 0.001 weight % or less than 0.0001 weight %aluminum or zirconium, relative to the total weight of the formulation.

The present disclosure provides antiperspirant products comprising as an antiperspirant active a complex of a quaternary ammonium polymer (such as polyacrylamidopropyltrimonium chloride) with a polyphosphate, e.g., any of the quaternary ammonium polymer (such as polyacrylamidopropyltrimonium chloride)/ polyphosphate complexes discussed herein, as well as methods of making and using such products. The present disclosure further provides methods of reducing sweat comprising applying the composition to skin, and methods of killing bacteria comprising contacting the bacteria with the composition.

In one embodiment, the present disclosure provides a composition (Composition 1), comprising at least one quaternary ammonium polymer and at least one polyphosphate having 2 or more phosphate units, wherein the composition comprises from 1-50 wt% of polyphosphate by weight of the composition, wherein the ratio of polyphosphate to polymer is from 1:2 to 50:1 by weight, wherein the least one quaternary ammonium polymer and at least one polyphosphate having 2 or more phosphate units form a soluble complex, and wherein a solution of the composition forms a precipitate upon dilution with water.
The quaternary ammonium polymer can be a poly (N-trialkylaminoalkyl)acrylamide.
The quaternary ammonium polymer can be polyacrylamidopropyltrimonium chloride.
The polyacrylamidopropyltrimonium chloride can have a number average molecular weight 100,000 to 500,000 daltons, optionally 200,000 to 400,000 daltons, or about 300,000 daltons.
The composition can be in an aqueous solvent.
The polyphosphate can have 3 or more phosphate units, e.g., 4 or more phosphate units, or 6 or more phosphate units.
The polyphosphate can have from 6 to 50 phosphate units, e.g., 6 to 30 phosphate units, or 10 to 20 phosphate units, or about 23 phosphate units.
The polyphosphate can comprise sodium tripolyphosphate.
The polyphosphate can comprise a long-chain polyphosphate, e.g., a polyphosphate having an average of 6, 9, 11, 12, 13, 15, 17, 18, 19, 21, 23, 24 or 25 phosphate units.
The polyphosphate can comprise a long-chain polyphosphate having an average length of 20-25 phosphate units, e.g., about 23 phosphate units.
The polyphosphate can have a P₂O₅ content of 68-71 wt%, e.g., about 69 wt%.
The polyphosphate can comprise sodium polymetaphosphate.
The ratio of polyphosphate to polymer can be for example, 1:1 to 10:1, or 1:1 to 9:1, or 1:1 to 8:1, or 2:1 to 8:1, or 2:1 to 6:1, or 2:1 to 5:1, or 2:1 to 4:1, or 2:1 to 3:1, or 10:1 to 50:1, or 10:1 to 40:1 or 10:1 to 25:1 or 10:1 to 25:1, or 12:1 to 24:1, or 16:1 to 20:1, or about 2:1, or about 2.5:1 or about 3:1, or about 12:1 or about 16:1 or about 20:1, by weight.
The composition can comprise e.g., from 1-40%, from 1-30%, from 2-20%, from 2-10%, from 5-40%, from 5-30%, from 5-20%, from 10-50%, from 10-40%, from 10-30%, from 10-20%, from 20-50%, from 20-40%, from 20-30%, from 30-50%, from 30-40%, or from 5-10%, or from 1-10%, or from 1-5% polyphosphate, by weight of the composition.
The composition can be a personal care composition, optionally, at least one of an antiperspirant or a deodorant.
The composition can be substantially free of aluminum complexes, or can be substantially free of zirconium complexes, or can be substantially free of both aluminum and zirconium complexes.
A method of occluding the pores of a subject comprises applying any composition described herein, wherein the composition can be a personal care composition, to the skin of the subject.
Further disclosed is the use of the any composition described herein, for the occlusion of the pores of a subject.
Further disclosed is the use of a soluble complex comprising at least one quaternary ammonium polymer and at least one polyphosphate having 2 or more phosphate units for the occlusion of the pores of a subject.
The complex can precipitate out of aqueous solution to occlude the pores.

Optional ingredients that can be included in an antiperspirant and/or deodorant formulation of the compositions of the present disclosure include solvents; water-soluble alcohols such as C₂₋₈ alcohols including ethanol; glycols including propylene glycol, dipropylene glycol, tripropylene glycol and mixtures thereof; glycerides including mono-, di- and triglycerides; medium to long chain organic acids, alcohols and esters; surfactants including emulsifying and dispersing agents; amino acids including glycine; structurants including thickeners and gelling agents, for example polymers, silicates and silicon dioxide; emollients; fragrances; and colorants including dyes and pigments. If desired, an antiperspirant and/or deodorant agent additional to the soluble quaternary ammonium polymer polyphosphate complex can be included.

The antiperspirant compositions can be formulated into topical antiperspirant and/or deodorant formulations suitable for application to skin, illustratively a stick, a gel, a cream, a roll-on, a soft solid, a powder, a liquid, an emulsion, a suspension, a dispersion or a spray. The composition can comprise a single phase or can be a multi-phase system, for example a system comprising a polar phase and an oil phase, optionally in the form of a stable emulsion. The composition can be liquid, semi-solid or solid. The antiperspirant and/or deodorant formulation can be provided in any suitable container such as an aerosol can, tube or container with a porous cap, roll-on container, bottle, container with an open end, etc.

The compositions can be used in a method to reduce sweating by applying the composition to skin. In certain embodiments, the application is to axilla. Thus the present disclosure provides a method for controlling perspiration comprising applying to skin an antiperspirant effective amount of a formulation of any embodiment embraced or specifically described herein.

The present invention is further illustrated through the following non-limiting example(s).

### EXAMPLES

### Example 1

Sodium tripolyphosphate (STPP) is prepared as 10% and 15% by weight solutions in deionized water. These solutions are mixed in a 1:1:1 weight ratio with polyacrylamidopropyltrimonium chloride solution (provided as a 20% active solution as N-Durance 1000 from Ashland Chemical with a weight average molecular weight of about 300,000) and deionized water. To avoid precipitating too early, the polymer solution is added last to the mixture. If added earlier, the low concentration of the polymer in the STPP/water solution will cause the polymer to precipitate out. In this example, the STPP is added to the polymer and then the water is added. This results in clear solutions. The prepared solutions are shown in Table 1 below.

**Table 1**

| Example No. | Mass STPP solution (g) | Mass Water (g) | Mass 20% polymer solution (g) | Final % STPP | Final % polymer | pH after prep. |
|---|---|---|---|---|---|---|
| 15% STPP, 20% polymer water | 7 | 7.3 | 7 | 4.92 | 6.6 | 7.93 |
| 10% STPP, 20% polymer water | 7.1 | 7 | 7 | 3.37 | 6.6 | 7.84 |

The solutions are clear.

### Example 2

The solutions from Example 1 are diluted 2x, 4x, 8x, 16x, and 32x with deionized water. After dilution, percent transmittance of the samples is measured on a Turbiscan instrument. For the 15% STPP solution, the 8x, 16x, and 32x dilutions show immediate precipitation with about 2% transmittance. In contrast, the 2x and 4x dilutions show some initial precipitation with about 65% transmittance. For the 10% STPP solution, results are similar: the 8x, 16x, and 32x dilutions show immediate precipitation with about 2% transmittance, and the 2x and 4x dilutions show some precipitation with about 65-67% transmittance. The pH of the solutions 30 minutes after dilution is also measured. The results are shown in Table 2 below.

**Table 2**

| Dilution | pH of 15% solution | pH of 10% solution |
|---|---|---|
| 2x | 8.21 | 8.1 |
| 4x | 8.43 | 8.33 |
| 8x | 8.63 | 8.45 |
| 16x | 8.78 | 8.6 |
| 32x | 8.85 | 8.61 |

### Example 3

Examples 1 and 2 are repeated with a lower concentration of polymer by using a 2.5 weight % solution of polyacrylamidopropyltrimonium chloride, instead of the 20 weight % solution in Example 1 and the elimination of the additional water. The STPP and polymer solutions are mixed at a 1:1 weight ratio. The prepared solutions are shown in Table 3 below.

**Table 3**

| Example No. | Mass STPP solution (g) | Mass 2.5% polymer solution (g) | Final % STPP | Final % polymer | pH after prep. |
|---|---|---|---|---|---|
| 15% STPP, 2.5% polymer water | 10 | 10 | 7.3 | 1.25 | 8.54 |
| 10% STPP, 2.5% polymer water | 10 | 10 | 5 | 1.25 | 8.75 |

The solutions are clear. The solutions are diluted 2x, 4x, 8x, 16x, and 32x with deionized water. After dilution, percent transmittance of the samples is measured on a Turbiscan instrument. For the 15% STPP solution, the 8x, 16x, and 32x dilutions again show immediate precipitation. For the 10% STPP solution, the results are similar: the 4x, 8x, 16x, and 32x dilutions show immediate precipitation.

### Comparative Example 1

Solutions of 15% STPP, 10% STPP, 2.5% polymer, and 1.25% polymer are prepared. The samples are diluted 2x, 4x, 8x, 16x, and 32x with deionized water. All samples remain clear after dilution. This shows that a complex is formed between the polymer and the polyphosphate.

### Example 4

Sodium polymetaphosphate (SPMP) solutions are prepared as 10%, 20%, 40%, 50%, and 60% by weight solutions in deionized water. These solutions are mixed in a 1:1:1 weight ratio with polyacrylamidopropyltrimonium chloride solution (provided as a 20% active solution) and deionized water. To avoid precipitating too early, the polymer solution is added last to the mixture. If added earlier, the low concentration of the polymer in the SPMP/water solution will cause the polymer to precipitate out. In this example, the SPMP is added to the polymer and then the water is added. This results in clear solutions. The prepared solutions are shown in Table 4 below.

**Table 4**

| Example No. | Mass SPMP solution (g) | Mass Water (g) | Mass 20% polymer solution (g) | Final % SPMP | Final % polymer | pH after prep. |
|---|---|---|---|---|---|---|
| 60% SPMP, 20% polymer water | 11.8 | 11.8 | 11.8 | 19.9 | 6.7 | 4.69 |
| 50% SPMP, 20% polymer water | 16 | 16 | 16 | 16.7 | 6.7 | 4.67 |
| 40% SPMP, 20% polymer water | 16 | 16 | 16 | 13.3 | 6.7 | 4.63 |
| 20% SPMP, 20% polymer water | 16 | 16 | 16 | -- | 6.7 | 4.47 |
| 10% SPMP, 20% polymer water | 16 | 16 | 16 | -- | 6.7 | 4.32 |

The 40%, 50%, and 60% solutions as prepared are clear, but a precipitate is present during formation of the 10% and 20% solutions.

### Example 5

The 40%, 50%, and 60% solutions from Example 4 are diluted 2x, 4x, 8x, and 16x with deionized water. After dilution, percent transmittance of the samples is measured on a Turbiscan instrument. For the 40% solution, all dilutions show immediate precipitation with about 4-7% transmittance. For the 50% solution, all dilutions show immediate precipitation with about 2-7% transmittance. For the 60% solution, all dilutions show immediate precipitation with about 3-5% transmittance. The pH of the solutions 30 minutes after dilution is also measured. The results are in Table 5 below.

**Table 5**

| Dilution | pH of 40% solution | pH of 50% solution | pH of 50% solution |
|---|---|---|---|
| 2x | 4.76 | 4.76 | 4.84 |
| 4x | 4.95 | 5 | 5.05 |
| 8x | 5.16 | 5.21 | 5.29 |
| 16x | 5.42 | 5.42 | 5.55 |

### Example 6

Since perspiration is typically slightly acidic, it is important that the precipitates that form do not redissolve under acidic conditions. The 2x, 4x, 8x, and 16x dilutions of the 40%, 50%, and 60% SPMP solutions are tested for acid resistance. All solutions were treated with acid to lower the pH to about 1.5. It is found that the precipitate in each sample remained substantially undissolved at the lower pH.

### Example 7

Examples 4 and 5 are repeated using the 2.5 weight % solution of polymer, instead of the 20 weight % solution of polymer used in Example 4, and the elimination of the additional water. The SPMP and polymer solutions were mixed at 1:1 weight ratio. The prepared solutions are shown in Table 6 below.

**Table 6**

| Example No. | Mass SPMP solution (g) | Mass 2.5% polymer solution (g) | Final % SPMP | Final % polymer | pH after prep. |
|---|---|---|---|---|---|
| 60% SPMP, 2.5% polymer | 10 | 10 | 30 | 1.25 | 4.69 |
| 50% SPMP, 2.5 polymer | 10 | 10 | 25 | 1.25 | 4.82 |
| 40% SPMP, 2.5% polymer | 10 | 10 | 20 | 1.25 | 4.93 |
| 30 % SPMP, 2.5% polymer | 10 | 10 | 15 | 1.25 | 5.04 |
| 20% SPMP, 2.5% polymer | 10 | 10 | Turbid | | |
| 10% SPMP, 2.5% polymer | 10 | 10 | | | |

The 30%, 40%, 50%, and 60% solutions as prepared are clear, but a precipitate is present during formation of the 10% and 20% solutions.

The solutions are diluted 2x and 4x with deionized water. For the 2x dilution, the 30% and 40% solutions show immediate precipitation, but the 50% and 60% solutions do not. For the 4x dilution, all samples show immediate precipitation.

### Example 8

The SPMP/polyacrylamidopropyltrimonium chloride complex precipitate is analyzed with X-ray Photoelectric Spectroscopy by depositing the precipitate on a Silicon wafer and allowing the precipitate to dry into a film. The results are shown in Table 7 below.

**Table 7**

| Material | Atomic Percent | | | | | |
|---|---|---|---|---|---|---|
| | C | O | N | N⁺ | Na | P |
| SPMP | | 59.8 | | | 21.87 | 18.33 |
| Polyacrylamidopropyltrimonium chloride | 75 | 11.43 | 7.36 | 6.21 | | |
| Precipitate | 44.9 | 31.39 | 4.4 | 4.13 | 6.08 | 9.1 |

The analysis of the SPMP / polymer thin film precipitate reveals that all of the elements contained in both starting materials are present in the precipitate. This suggests that the precipitate is a combination of SPMP and polymer. The O/P ratio is slightly higher in the precipitate than in SPMP due to the additional oxygen from the polymer. The Na/P ratio is lower than that for SPMP, indicating a deficiency in sodium in the precipitate relative to SPMP. The ratio of (N⁺ + Na)/P is the same as that for the SPMP reference, indicating that the N⁺ from the polymer is substituting for Na in SPMP, which is theorized to constitute a bonding between the starting materials that forms the precipitate. The P/N⁺ ratio can be used to approximate the stoichiometry for the precipitate, since N⁺ is unique to the polymer while P is unique to the SPMP. This ratio indicates that for each SPMP unit of 25 P atoms that there are approximately 11 N⁺ atoms. Thus the precipitate is composed of approximately 11 polymer monomer units for each SPMP unit. About half the Na atoms in the SPMP are replaced by N⁺ from the polymer. The calculated composition for the 1 SPMP : 11 monomer structure is near to that for the actual precipitate. This suggests that the proposed stoichiometry for the precipitate approximates that for the actual precipitate.

### Example 9

Three aqueous solutions of SPMP are prepared: 10% SPMP, 20% SPMP, and 30% SPMP. Additionally, two solutions of polyacrylamidopropyltrimonium chloride are prepared: 2.5% polymer, and 1.25% polymer. All of these solutions remain clear after formation. The 10% SPMP and the 1.25% polymer solutions are each diluted 2x and 4x with deionized water and aged for three weeks. All dilutions remain clear after aging. This shows that the formation of a precipitate is unique to the combination of the polymer and the phosphate in the same solution, thus suggesting that a complex is formed between them that is responsible for the precipitation.

The above examples show that the polyphosphate/polymer complex can precipitate when diluted. When applied to skin, perspiration can cause the complex to form a precipitate, which can then occlude pores.

### Example 10

A typical roll-on antiperspirant formulation comprising the polyphosphate/polymer complex of the present disclosure can be formulated as described in Table 8 below:

**Table 8**

| Material | Weight Percent |
|---|---|
| Polyphosphate/polymer complex | 5-30% |
| Steareth-20 | 1-10%, e.g., 2% |
| PPG-15 Stearyl Ether | 1-10%, e.g, 2% |
| Steareth-2 | 1-15%, e.g., 3% |
| Cyclomethicone | 1-10%, e.g., 2% |
| Fragrance | 0.5-5%, e.g, 1% |
| Water | Q.S. |

## Claims

1. A composition, comprising at least one quaternary ammonium polymer and at least one polyphosphate having 2 or more phosphate units, wherein the composition comprises from 1-50 wt% of polyphosphate by weight of the composition, wherein the ratio of polyphosphate to polymer is from 1:2 to 50:1 by weight, wherein the least one quaternary ammonium polymer and at least one polyphosphate having 2 or more phosphate units form a soluble complex, and wherein a solution of the composition forms a precipitate upon dilution with water.

2. The composition of claim 1, wherein the quaternary ammonium polymer is a poly (N-trialkylaminoalkyl)acrylamide.

3. The composition of claim 2, wherein the quaternary ammonium polymer is polyacrylamidopropyltrimonium chloride, optionally wherein the polyacrylamidopropyltrimonium chloride has a number average molecular weight 100,000 to 500,000 daltons, optionally 200,000 to 400,000 daltons, or about 300,000 daltons.

4. The composition of any preceding claim, wherein the composition is in an aqueous solvent.

5. The composition of any preceding claim, wherein the polyphosphate has 3 or more phosphate units, e.g., 4 or more phosphate units, 6 or more phosphate units, or from 6 to 50 phosphate units, e.g., 6 to 30 phosphate units, or 10 to 20 phosphate units, or about 23 phosphate units.

6. The composition of any of claims 1-5, wherein the polyphosphate comprises sodium tripolyphosphate.

7. The composition of any of claim 1-6, wherein the polyphosphate comprises sodium polymetaphosphate.

8. The composition of any preceding claim, wherein the composition is a personal care composition, optionally, at least one of an antiperspirant or a deodorant.

9. The composition of any preceding claim, wherein the composition includes less than 2 weight% aluminum or zirconium.

10. A method of occluding the pores of a subject comprising applying the composition of any preceding claim, e.g., the personal care composition of any preceding claim, to the skin of the subject.

11. The use of the composition of any of claims 1-9 for the occlusion of the pores of a subject.

12. The use of a soluble complex comprising at least one quaternary ammonium polymer and at least one polyphosphate having 2 or more phosphate units for the occlusion of the pores of a subject.

13. The use of claim 12, wherein the complex precipitates out of aqueous solution to occlude the pores.

## Patentansprüche

1. Zusammensetzung umfassend mindestens ein quaternäres Ammoniumpolymer und mindestens ein Polyphosphat mit 2 oder mehr Phosphateinheiten, wobei die Zusammensetzung von 1-50 Gew.-% Polyphosphat, bezogen auf das Gewicht der Zusammensetzung, umfasst, wobei das Verhältnis von Polyphosphat zu Polymer von 1:2 bis 50:1, bezogen auf das Gewicht, beträgt, wobei das mindestens eine quaternäre Ammoniumpolymer und mindestens ein Polyphosphat mit 2 oder mehr Phosphateinheiten einen löslichen Komplex bilden, und wobei eine Lösung der Zusammensetzung bei Verdünnung mit Wasser ein Präzipitat bildet.

2. Zusammensetzung nach Anspruch 1, wobei das quaternäre Ammoniumpolymer ein Poly(N-trialkylaminoalkyl)acrylamid ist.

3. Zusammensetzung nach Anspruch 2, wobei es sich bei dem quaternären Ammoniumpolymer um Polyacrylamidopropyltrimoniumchlorid handelt, wobei das Polyacrylamidopropyltrimoniumchlorid gegebenenfalls ein zahlenmittleres Molekulargewicht von 100.000 bis 500.000 Dalton, gegebenenfalls 200.000 bis 400.000 Dalton oder etwa 300.000 Dalton aufweist.

4. Zusammensetzung nach einem beliebigen vorhergehenden Anspruch, wobei die Zusammensetzung in einem wässrigen Lösungsmittel vorliegt.

5. Zusammensetzung nach einem beliebigen vorhergehenden Anspruch, wobei das Polyphosphat 3 oder mehr Phosphateinheiten, z.B. 4 oder mehr Phosphateinheiten, 6 oder mehr Phosphateinheiten oder 6 bis 50 Phosphateinheiten, z.B. 6 bis 30 Phosphateinheiten oder 10 bis 20 Phosphateinheiten oder etwa 23 Phosphateinheiten aufweist.

6. Zusammensetzung nach einem beliebigen der Ansprüche 1 bis 5, wobei das Polyphosphat Natriumtripolyphosphat umfasst.

7. Zusammensetzung nach einem beliebigen der Ansprüche 1-6, wobei das Polyphosphat Natriumpolymetaphosphat umfasst.

8. Zusammensetzung nach einem beliebigen vorhergehenden Anspruch, wobei es sich bei der Zusammensetzung um eine Körperpflegezusammensetzung handelt, gegebenenfalls um zumindest eines von einem Antitranspirant oder einem Deodorant.

9. Zusammensetzung nach einem beliebigen vorhergehenden Anspruch, wobei die Zusammensetzung weniger als 2 Gew.-% Aluminium oder Zirkonium enthält.

10. Verfahren zum Verschließen der Poren einer Person, umfassend das Auftragen der Zusammensetzung nach einem beliebigen vorhergehenden Anspruch, z.B. der Körperpflegezusammensetzung nach einem beliebigen vorhergehenden Anspruch, auf die Haut der Person.

11. Verwendung der Zusammensetzung nach einem beliebigen der Ansprüche 1 bis 9 zum Verschließen der Poren eines Subjekts.

12. Verwendung eines löslichen Komplexes, der mindestens ein quaternäres Ammoniumpolymer und mindestens ein Polyphosphat mit 2 oder mehr Phosphateinheiten umfasst, zum Verschließen der Poren eines Subjekts.

13. Verwendung nach Anspruch 12, wobei der Komplex aus wässriger Lösung präzipitiert, um die Poren zu verschließen.

## Revendications

1. Composition, comprenant au moins un polymère d'ammonium quaternaire et au moins un polyphosphate ayant 2 motifs phosphate ou plus, dans laquelle la composition comprend de 1 à 50 % en poids de polyphosphate en poids de la composition, dans laquelle le rapport du polyphosphate au polymère est de 1:2 à 50:1 en poids, dans laquelle l'au moins un polymère d'ammonium quaternaire et l'au moins un polyphosphate ayant 2 motifs phosphate ou plus forment un complexe soluble, et dans laquelle une solution de la composition forme un précipité lors de la dilution avec de l'eau.

2. Composition selon la revendication 1, dans laquelle le polymère d'ammonium quaternaire est un poly(N-trialkylaminoalkyl)acrylamide.

3. Composition selon la revendication 2, dans laquelle le polymère d'ammonium quaternaire est le chlorure de polyacrylamidopropyltrimonium, éventuellement dans laquelle le chlorure de polyacrylamidopropyltrimonium a un poids moléculaire moyen en nombre de 100 000 à 500 000 daltons, éventuellement de 200 000 à 400 000 daltons, ou d'environ 300 000 daltons.

4. Composition selon l'une quelconque des revendications précédentes, dans laquelle la composition est dans un solvant aqueux.

5. Composition selon l'une quelconque des revendications précédentes, dans laquelle le polyphosphate a 3 motifs phosphate ou plus, par exemple 4 motifs phosphate ou plus, 6 motifs phosphate ou plus, ou de 6 à 50 motifs phosphate, par exemple 6 à 30 motifs phosphate, ou 10 à 20 motifs phosphate, ou environ 23 motifs phosphate.

6. Composition selon l'une quelconque des revendications 1 à 5, dans laquelle le polyphosphate comprend le tripolyphosphate de sodium.

7. Composition selon l'une quelconque des revendications 1 à 6, dans laquelle le polyphosphate comprend le polymétaphosphate de sodium.

8. Composition selon l'une quelconque des revendications précédentes, dans laquelle la composition est une composition de soins personnels, éventuellement, au moins l'un d'un anti-transpirant ou d'un déodorant.

9. Composition selon l'une quelconque des revendications précédentes, dans laquelle la composition comprend moins de 2 % en poids d'aluminium ou de zirconium.

10. Procédé d'occlusion des pores d'un sujet comprenant l'application de la composition selon l'une quelconque des revendications précédentes, par exemple, la composition de soins personnels selon l'une quelconque des revendications précédentes, sur la peau du sujet.

11. Utilisation de la composition selon l'une quelconque des revendications 1 à 9 pour l'occlusion des pores d'un sujet.

12. Utilisation d'un complexe soluble comprenant au moins un polymère d'ammonium quaternaire et au moins un polyphosphate ayant 2 motifs phosphate ou plus pour l'occlusion des pores d'un sujet.

13. Utilisation selon la revendication 12, dans laquelle le complexe précipite hors de la solution aqueuse pour occlure les pores.
